(19) Europäisches Patentamt
European Patent Office
Office européen des brevets

(11) Veröffentlichungsnummer: **0 283 777**
**A1**

(12) # EUROPÄISCHE PATENTANMELDUNG

(21) Anmeldenummer: **88103146.2**

(51) Int. Cl.⁴: **C07C 147/06**, A01N 41/10

(22) Anmeldetag: **02.03.88**

(30) Priorität: **14.03.87 DE 3708320**

(43) Veröffentlichungstag der Anmeldung: **28.09.88 Patentblatt 88/39**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Fest, Christa, Dr.**
**Im Johannistal**
**D-5600 Wuppertal(DE)**
Erfinder: **Findeisen, Kurt, Dr.**
**In der Follmühle 10**
**D-5068 Odenthal 2(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**
Erfinder: **Reinecke, Paul, Dr.**
**Steinstrasse 8**
**D-5090 Leverkusen 3(DE)**

(54) **Benzaldoxim-Derivate.**

(57) Benzaldoxim-Derivate der Formel (I)

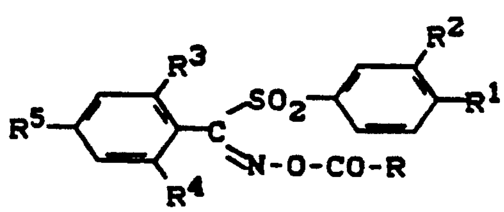

(I)

in welcher
R bis $R^5$ die in der Beschreibung angegebenen Bedeutungen haben und ihre Verwendung in Schädlingsbekämpfungsmitteln.

Die neuen Benzaldoxim-Derivate der allgemeinen Formel (I) können aus geeigneten Phenylsulfonyl-benzaldoximen mit geeigneten Carbonylverbindungen hergestellt werden.

EP 0 283 777 A1

## Benzaldoxim-Derivate

Die vorliegende Erfindung betrifft neue Benzaldoxim-Derivate, ein Verfahren zu ihrer Herstellung und ihre Verwendung als Schädlingsbekämpfungsmittel, insbesondere als Fungizide.

Es sind bereits eine Reihe von Aldoxim-Derivaten bekannt. So z.B. Arylsulfonylbenzaldoxime, wie das α-Phenylsulfonyl-2,6-dichlor-benzaldoxim, und ihre Verwendung als Schädlingsbekämpfungsmittel, vor allem ihre Verwendung in Mitteln zur Bekämpfung von Weizensteinbrand, ist bekannt (vgl. Schweizer Patent Nr. 423.350). Weiterhin sind Phenyl-pyridinaldoxime, wie z.B. das Phenyl-O-ethylcarbonyl-pyridinaldoxim, und ihre Antihistamin-Wirkung bekannt (vgl. J. Pharm. Sci. 56(1967) Nr. 10, S. 1354-1357).

Es wurden neuen Benzaldoxim-Derivate der allgemeinen Formel (I)

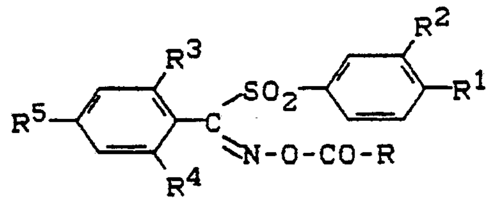

(I)

in welcher

R für Alkyl, Alkoxy, Halogenalkyl, Halogenalkoxy, Alkenyloxy, gegebenenfalls jeweils ein-bis mehrfach, gleich oder verschieden substituiertes Aryl oder Aryloxy, gegebenenfalls ein-bis mehrfach, gleich oder verschieden im Arylrest substituiertes Aralkyloxy, für gegebenenfalls ein-bis mehrfach, gleich oder verschieden substituiertes Cycloalkyloxy oder für einen gegebenenfalls ein-bis mehrfach, gleich oder verschieden substituierten Heterocyclus steht,

$R^1$ für Wasserstoff, Halogen, Alkyl oder Alkoxy steht,

$R^2$ für Wasserstoff, Alkyl oder Halogenalkyl steht,

$R^3$ für Wasserstoff oder Halogen steht,

$R^4$ für Halogen steht und

$R^5$ für Wasserstoff oder Halogen steht, gefunden.

Weiterhin wurde gefunden, daß man die Benzaldoxim-Derivate der Formel (I)

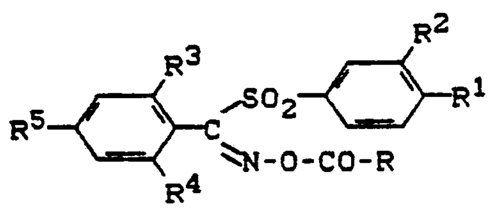

(I)

in welcher

R für Alkyl, Alkoxy, Halogenalkyl, Halogenalkoxy, Alkenyloxy, gegebenenfalls jeweils ein-bis mehrfach, gleich oder verschieden substituiertes Aryl oder Aryloxy, gegebenenfalls ein-bis mehrfach, gleich oder verschieden im Arylrest substituiertes Aralkyloxy, für gegebenenfalls ein-bis mehrfach, gleich oder verschieden substituiertes Cycloalkyloxy oder für einen gegebenenfalls ein-bis mehrfach, gleich oder verschieden substituierten Heterocyclus steht,

$R^1$ für Wasserstoff, Halogen, Alkyl oder Alkxoy steht,

$R^2$ für Wasserstoff, Alkyl oder Halogenalkyl steht,

$R^3$ für Wasserstoff oder Halogen steht,

$R^4$ für Halogen steht und

$R^5$ für Wasserstoff oder Halogen steht,

erhält, wenn man

Phenylsulfonyl-benzaldoxime der allgemeinen Formel (II)

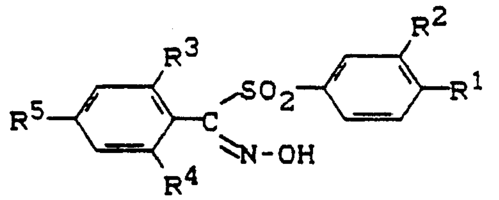

(II)

in welcher
$R^1$, $R^2$, $R^3$, $R^4$ und $R^5$ die oben angegebenen Bedeutungen haben,
mit Carbonylverbindungen der allgemeinen Formel (III)

X - CO - R (III)

in welcher
R die oben angegebenen Bedeutungen hat und
X für ein Halogenatom, vorzugsweise Chlor, oder für den Rest -O-COR steht, worin R die oben angegebene Bedeutung hat,
gegebenenfalls in Gegenwart eines Lösungs-oder Verdünnungsmittels und gegebenenfalls in Gegenwart eines Säurebindemittels umsetzt.

Die erfindungsgemäßen Benzaldoxim-Derivate der Formel (I) weisen starke biologische, vor allem fungizide Eigenschaften auf.

Überraschenderweise zeigen dabei die erfindungsgemäßen Verbindungen eine erheblich höhere vor allem fungizide Wirksamkeit als die aus dem Stand der Technik bekannten Verbindungen, die strukturell und/oder wirkungsmäßig sehr naheliegende Verbindungen sind.

Die erfindungsgemäßen Verbindungen der Formel (I) können als syn-oder anti-Isomere oder als deren Gemische in unterschiedlicher Zusammensetzung anfallen. Die Erfindung bezieht sich sowohl auf die reinen Isomeren als auch auf die Isomerengemische.

Die Alkylreste R, $R^1$ und $R^2$ sowie die Alkylteile in den Alkoxy-Resten in R und $R^1$ können geradkettig oder verzweigt sein und enthalten vorzugsweise jeweils 1 bis 6, insbesondere 1 bis 4 Kohlenstoffatome. Beispielhaft seien genannt: Methyl, Ethyl, n-Propyl, iso-Propyl, n-Butyl, sec.-Butyl, iso-Butyl, tert.-Butyl, n-Pentyl, iso-Pentyl, sec.-Pentyl, n-Hexyl, sec. -Hexyl, Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, n-Hexoxy und sec.-Hexoxy.

Der Alkenyloxyrest in R enthält vorzugsweise 2 bis 6, insbesondere 2 bis 5, besonders bevorzugt 2 oder 3 Kohlenstoffatome. Beispielhaft seien genannt: Vinyl, Allyl, Propenyl(1), Butenyl und Pentenyl.

Die Halogenalkylteile in R und $R^2$ in den Resten Halogenalkyl und Halogenalkoxy enthalten vorzugsweise jeweils 1 bis 6, insbesondere 1 bis 4, besonders bevorzugt 1 oder 2 Kohlenstoffatome und vorzugsweise 1 bis 9, insbesondere 1 bis 5, besonders bevorzugt 1 bis 4 gleiche oder verschiedene Halogenatome. Beispielhaft seien genannt: Monochlormethyl, Trichlormethyl, Trifluormethyl, Dichlorfluormethyl, Trichlorethyl, Tetrachlorethyl, Trichlormethoxy, Trichlorethoxy und Tetrachlorethoxy.

Halogen in R bis $R^5$ auch in den Resten wie Halogenalkyl, Halogenalkoxy oder in den Arylsubstituenten u.s.w. bedeutet Fluor, Chlor, Brom oder Iod, insbesondere Fluor oder Chlor, wenn an anderer Stelle nicht besonders definiert.

Aryl auch im Aryloxyrest in R kann für einen aromatischen Kohlenwasserstoffrest mit 6 bis 10 Kohlenstoffatomen stehen. Beispielsweise seien Phenyl und Naphthyl genannt. Bevorzugt ist Phenyl.

Aralkyl im Aralkoxy in R kann für einen Rest mit 7 bis 18 Kohlenstoffatomen stehen, wobei ein geradkettiger oder verzweigter Alkylrest ($C^1$ bis $C^6$) durch einen aro matischen Rest ($C^6$ bis $C^1{}_0$) substituiert sein kann. Beispielsweise seien Benzyl, Phenyl-ethyl und Phenyl-propyl genannt. Bevorzugt ist Benzyl.

Cycloalkyl im Cycloalkyloxy kann für einen cyclischen, bevorzugt monocyclischen Kohlenwasserstoffrest mit 5 bis 7 Kohlenstoffatomen stehen. Beispielsweise seien Cyclopentyl, Cyclohexyl und Cycloheptyl genannt. Bevorzugt sind Cyclopentyl und Cyclohexyl.

Heterocyclus kann für einen gesättigten, teilweise oder ganz ungesättigten 5-bis 8-gliedrigen Ring, vorzugsweise 5-oder 6-gliedrigen Ring mit 1 bis 3, vorzugsweise 1 oder 2, insbesondere 1 Heteroatom stehen, wobei als Heteroatome Schwefel, Sauerstoff oder Stickstoff, insbesondere Schwefel genannt seien.

Das genannte Aryl, Aryloxy und Aralkyloxy kann unsubstituiert oder substituiert sein; als Substituenten kommen 1 bis 5, bevorzugt 1 bis 3 Substituenten, besonders bevorzugt 1 oder 2 Substituenten aus der Gruppe der niederen Alkylgruppen mit 1 bis 4 Kohlenstoffatomen (Methyl, Ethyl, Propyl, Isopropyl, Butyl, Isobutyl), der niederen Alkoxygruppen mit 1 bis 4 Kohlenstoffatomen (Methoxy, Ethoxy, Propoxy, Isopropoxy, Butoxy, Isobutoxy) oder der Halogene (Fluor, Chlor, Brom) in Frage. Weitere Substituenten können sein: die Nitrogruppe sowie die Acetylgruppe.

Das genannte Cycloalkyloxy kann unsubstituiert oder substituiert sein; als Substituenten kommen 1 bis 5, bevorzugt 1 bis 3, besonders bevorzugt 1 oder 3 Substituenten aus der Gruppe der niederen Alkylgruppen mit 1 bis 4 Kohlenstoffatomen (Methyl, Ethyl, Propyl, i-Propyl, n-und s-Butyl, i-Butyl, t-Butyl) infrage.

Der genannte Heterocyclus kann unsubstituiert oder substituiert sein. Als Substituenten kommen 1 bis 3, bevorzugt 1 Substituent aus der Gruppe der niederen Alkyle mit 1 bis 4 Kohlenstoffatomen, wie oben aufgezählt, infrage.

Die erfindungsgemäßen Benzaldoxim-Derivate sind durch die Formel (I) allgemein definiert. Bevorzugt sind Verbindungen der Formel (I),

in welcher

R für geradkettiges oder verzweigtes Alkyl mit 1 bis 6 Kohlenstoffatomen, für geradkettiges oder verzweigtes Alkoxy mit 1 bis 6 Kohlenstoffatomen, für Halogenalkyl oder Halogenalkoxy mit jeweils 1 bis 6 Kohlenstoffatomen im geradkettigen oder verzweigten Halogenalkylrest und 1 bis 9 gleichen oder verschiedenen Halogenatomen, für geradkettiges oder verzweigtes Alkenyloxy mit 2 bis 6 Kohlenstoffatomen, für unsubstituiertes oder jeweils ein-bis fünffach, gleich oder verschieden durch geradkettiges oder verzweigtes Alkyl mit 1 bis 4 Kohlen stoffatomen, geradkettiges oder verzweigtes Alkoxy mit 1 bis 4 Kohlenstoffatomen, Halogen, Nitro und Acetyl substituiertes Aryl oder Aryloxy mit 6 bis 10 Kohlenstoffatomen, für unsubstituiertes oder ein-bis fünffach, gleich oder verschieden durch geradkettiges oder verzweigtes Alkyl oder Alkoxy mit 1 bis 4 Kohlenstoffatomen, Halogen, Nitro und Acetyl substituiertes Aralkyloxy mit 6 bis 12 Kohlenstoffatomen im Arylrest und 1 bis 6 Kohlenstoffatomen im geradkettigen oder verzweigten Alkylrest, für unsubstituiertes oder ein-bis fünffach, gleich oder verschieden durch geradkettiges oder verzweigtes Alkyl mit 1 bis 4 Kohlenstoffatmen substituiertes Cycloalkyloxy mit 5 bis 7 Kohlenstoffatomen im Cycloalkylteil oder für einen unsubstituierten oder ein-bis dreifach, gleich oder verschieden durch geradkettiges oder verzweigtes Alkyl mit 1 bis 4 Kohlenstoffatomen substituierten, gesättigten, teilweise oder ganz ungesättigten Heterocyclus mit 5-bis 8-Ringgliedern steht, der 1 bis 3 Heteroatome enthalten kann;

$R^1$ für Wasserstoff, Halogen, geradkettiges oder verzweigtes Alkyl mit 1 bis 6 Kohlenstoffatomen oder geradkettiges oder verzweigtes Alkoxy mit 1 bis 6 Kohlenstoffatomen steht,

$R^2$ für Wasserstoff, geradkettiges oder verzweigtes Alkyl mit 1 bis 6 Kohlenstoffatomen oder für geradkettiges oder verzweigtes Halogenalkyl mit 1 bis 6 Kohlenstoffatomen und 1 bis 9 gleichen oder verschiedenen Halogenatomen steht,

$R^3$ für Wasserstoff oder Halogen steht,

$R^4$ für Halogen steht und

$R^5$ für Wasserstoff oder Halogen steht.

Besonders bevorzugt sind Verbindungen der Formel (I),

in welcher

R für geradkettiges oder verzweigtes Alkyl mit 1 bis 4 Kohlenstoffatomen, für geradkettiges oder verzweigtes Alkoxy mit 1 bis 4 Kohlenstoffatomen, für Halogenalkyl oder Halogenalkoxy mit jeweils 1 bis 4 Kohlenstoffatomen im geradkettigen oder verzweigten Halogenalkylrest und 1 bis 5 gleichen oder verschiedenen Halogenatomen, für geradkettiges oder verzweigtes Alkenyloxy mit 2 bis 5 Kohlenstoffatomen, für unsubstituiertes oder jeweils ein-bis fünffach, gleich oder verschieden durch geradkettiges oder verzweigtes Alkyl mit 1 bis 3 Kohlenstoffatomen, geradkettiges oder verzweigtes Alkoxy mit 1 bis 3 Kohlenstoffatomen, Halogen, Nitro und Acetyl substituiertes Phenyl oder Phenoxy, für unsubstituiertes oder ein-bis dreifach, gleich oder verschieden durch geradkettiges oder verzweigtes Alkyl oder Alkoxy mit 1 bis 3 Kohlenstoffatomen, Halogen, Nitro und Acetyl substituiertes Benzyloxy, Phenethyloxy oder Phenylpropyloxy, oder für unsubstituiertes oder ein-bis dreifach, gleich oder verschieden durch geradkettiges oder verzweigtes Alkyl mit 1 bis 3 Kohlenstoffatomen substituiertes Cycloalkyloxy mit 5 bis 7 Kohlenstoffatomen im Cycloalkylteil oder für einen unsubstituierten oder einfach durch geradkettiges oder verzweigtes Alkyl mit 1 bis 3 Kohlenstoffatomen substituierten, gesättigten, teilweise gesättigten oder ungesättigten Heterocyclus mit 5 bis 7 Ringgliedern steht, der 1 oder 2 Schwefel-, Stickstoff-und/oder Sauerstoffatome enthalten kann;

$R^1$ für Wasserstoff, Halogen, geradkettiges oder verzweigtes Alkyl mit 1 bis 4 Kohlenstoffatomen oder geradkettiges oder verzweigtes Alkoxy mit 1 bis 4 Kohlenstoffatomen steht,

$R^2$ für Wasserstoff, geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 5 gleichen oder verschiedenen Halogenatomen steht,

$R^3$ für Wasserstoff oder Halogen steht,

$R^4$ für Halogen steht und
$R^5$ für Wasserstoff oder Halogen steht.

Ganz besonders bevorzugt sind Verbindungen der Formel (I), in welcher
R für geradkettiges oder verzweigtes Alkyl mit 1 bis 6 Kohlenstoffatomen, für geradkettiges oder verzweigtes Alkoxy mit 1 bis 6 Kohlenstoffatomen, für Halogenalkyl oder Halogenalkoxy mit jeweils 1 oder 2 Kohlenstoffatomen im geradkettigen oder verzweigten Halogenalkylrest und 1 bis 4 gleichen oder verschiedenen Halogenatomen, für geradkettiges oder verzweigtes Alkenyloxy mit 2 oder 3 Kohlenstoffatomen, für unsubstituiertes oder jeweils ein-oder zweifach, gleich oder verschieden durch Alkyl mit 1 oder 2 Kohlenstoffatomen, Alkoxy mit 1 oder 2 Kohlenstoffatomen, Halogen, Nitro und Acetyl substituiertes Phenyl oder Phenoxy, für unsubstituiertes oder ein-bis dreifach, gleich oder verschieden durch Alkyl oder Alkoxy mit 1 oder 2 Kohlenstoffatomen oder Halogen substituiertes Benzyl, für unsubstituiertes oder ein-bis dreifach, gleich oder verschieden durch Alkyl mit 1 oder 2 Kohlenstoffatomen substituiertes Cycloalkyloxy mit 5 oder 6 Kohlenstoffatomen im Cycloalkylteil oder für einen unsubstituierten oder ein-oder zweifach, gleich oder verschieden durch Alkyl mit 1 oder 2 Kohlenstoffatomen substituierten, ungesättigten Heterocyclus mit 5 oder 6 Ringgliedern steht, der Stickstoff-oder ein Schwefelatom enthalten kann;
$R^1$ für Wasserstoff, Halogen, Alkyl mit 1 oder 2 Kohlenstoffatomen oder Alkoxy mit 1 oder 2 Kohlenstoffatomen steht,
$R^2$ für Wasserstoff, Alkyl mit 1 oder 2 Kohlenstoffatomen oder für Halogenalkyl mit 1 oder 2 Kohlenstoffatomen und 1 bis 3 gleichen oder verschiedenen Halogenatomen steht,
$R^3$ für Wasserstoff oder Halogen steht,
$R^4$ für Halogen steht und
$R^5$ für Wasserstoff oder Halogen steht.

Insbesondere seien Verbindungen der Formel (I) genannt, in welcher
R für Methyl, Ethyl, Methoxy, Ethoxy, n-Propoxy, iso-Propoxy, iso-Butoxy, Allyloxy, 2,2,2-Trichlorethoxy, Benzyloxy, Chlormethyl, Phenyl, 2,6-Dichlorphenyl, 2,4-Dichlorphenyl, Phenoxy, Thienyl oder Cyclohexyloxy steht,
$R^1$ für Wasserstoff, Fluor, Chlor, Methyl oder Methoxy steht,
$R^2$ für Wasserstoff oder Trifluormethyl steht,
$R^3$ für Wasserstoff, Fluor oder Chlor steht,
$R^4$ für Fluor oder Chlor steht und
$R^5$ für Wasserstoff oder Chlor steht.

Verwendet man 2,6-Difluor-α-(4-methylphenylsulfonyl)-benzaldoxim und Acetanhydrid als Ausgangsverbindungen, so kann der Reaktionsablauf des erfindungsgemäßen Verfahrens durch das folgende Reaktionsschema wiedergegeben werden:

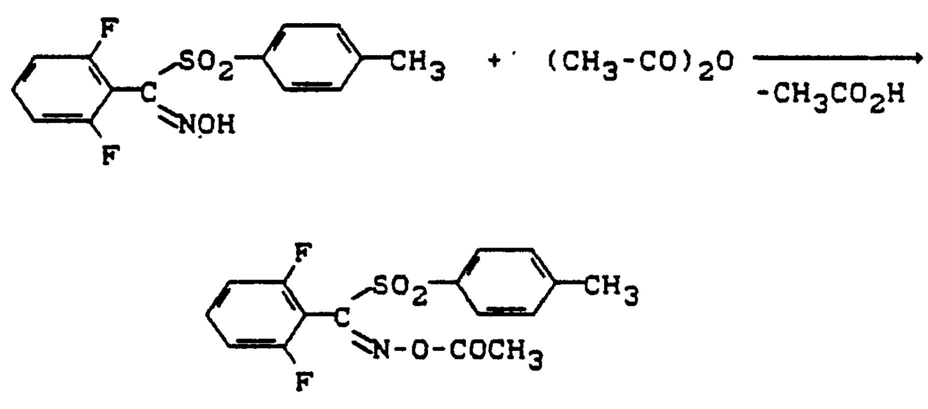


Die zur Durchführung des erfindungsgemäßen Verfahrens als Ausgangsstoffe benötigten Phenylsulfonyl-benzaldoxime der allgemeinen Formel (II) sind teilweise bekannt. Die bekannten und die neuen Verbindungen können nach Analogieverfahren hergestellt werden, z.B. nach dem im Schweizer Patent Nr. 423.350 im Beispiel VII beschriebenen Verfahren, indem man α-Halogen-benzaldoxime der Formel (IV)

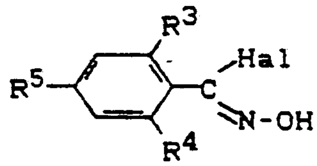

(IV)

worin
$R^3$, $R^4$ und $R^5$ die oben angegebene Bedeutung haben und
Hal für Halogen, vorzugsweise für Chlor steht, mit Benzolsulfinsäuren der Formel (V)

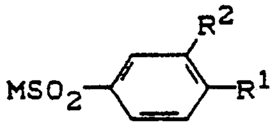

(V)

in welcher
R' und $R^2$ die angegebenen Bedeutungen haben und
M für Wasserstoff oder ein Alkalimetalläquivalent steht,
gegebenenfalls in Gegenwart eines Lösungsmittels und gegebenenfalls in Gegenwart eines Säureakzeptors umsetzt.

Die Sulfinsäuren sind bekannten Verbindungen.

Die weiterhin als Ausgangsstoffe benötigten Carbonylverbindungen sind durch die Formel (III) definiert. Es handelt sich um bekannte Verbindungen der organischen Chemie.

Das erfindungsgemäße Verfahren kann gegebenenfalls in Gegenwart eines Lösungs-oder Verdünnungsmittels durchgeführt werden. Als solche kommen prinzipiell alle inerten organischen Lösungsmittel infrage. Bevorzugt verwendet man Kohlenwasserstoffe, gegebenenfalls chlorierte, wie z.B. Benzol, Toluol, Xylol, Methylenchlorid, Chloroform, Tetrachlorkohlenstoff, Chlorbenzol und Dichlorbenzol, ferner Ether, wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid.

Als Säurebindemittel kommen für das erfindungsgemäße Verfahren übliche anorganische oder organische Säurebinder infrage. Als solche seien genannt: z.B. tert. Amine, wie Triethylamin, Pyridin, Triethylendiamin u.a.

Die Reaktionstemperatur des erfindungsgemäßen Verfahrens kann in einem größeren Temperaturbereich variiert werden. Im allgemeinen arbeitet man zwischen 0°C und 120°C, vorzugsweise zwischen 20°C und 70°C.

Die Reaktion wird normalerweise unter Normaldruck ausgeführt. Bei der Durchführung des erfindungsgemäßen Verfahrens legt man im allgemeinen die Verbindungen der Formel (II) in einem Lösungsmittel mit äquimolaren Mengen des Säurebinders vor und gibt die Carbonylverbindung der Formel (III) vorzugsweise ebenfalls in äquimolaren Mengen zu. Die Aufarbeitung erfolgt nach allgemein üblichen Methoden.

Eine besondere Ausführungsform ist noch zu erwähnen. Wenn X die Bedeutung -O-COR hat, d.h. also Carbonsäureanhydride der Formel (III) eingesetzt werden, arbeitet man ohne Lösungsmittel mit einem hohen Überschuß des Anhydrids, das dann gleichzeitig als Ausgangsstoff und Lösungsmittel dient. Die Aufarbeitung erfolgt ebenfalls nach üblichen Verfahren.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch als Schädlingsbekämpfungsmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;

Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier-und/oder - schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergier mittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe, wie Salze von Eisen, Mangan, Bor, Kuper, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die erfindungsgemäßen Verbindungen der Formel (I) zeigen eine besonders gute Wirkung gegen Erreger von Gemüse-und Obstkrankheiten, von Getreideerkrankungen und Reiskrankheiten, genannt seien Venturia-Arten, Phytophthora infestans, Leptosphaeria modorum, Pyricularia oryzae, ferner seien erwähnt Pyrenophora teres, Cochliobolus sativus und Puccinia recondita, bei entsprechender Konzentration ist auch eine in vitro-Wirkung gegen Bakterien zu verzeichnen.

In entsprechenden Konzentrationen zeigen die erfindungsgemäßen Stoffe auch herbizide Wirkungen.

## Anwendungsbeispiele:

In den folgenden Anwendungsbeispielen wurde die nachstehend aufgeführte Verbindung als Vergleichssubstanz eingesetzt:

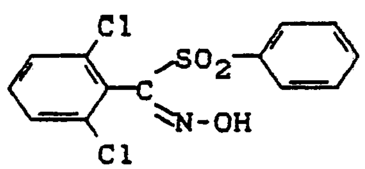

α-Benzolsulfonyl-2,6-dichlorbenzaldoxim (bekannt aus CH 423.350; Beispiel VII).

## Beispiel A

Pyricularia-Test (Reis) protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutlische Überlegenheit in der Wirksamkeit gegenüber dem Stand der Technik zeigen in diesem Test die erfindungsgemäßen Verbindungen bei z.B. einer Wirkstoffkonzentration von 0,025 %.

Beispiel B

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Hertellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Lufefeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen die meisten der erfindungsgemäßen Verbindungen bei einer Wirkstoffkonzentration von 0,025 Gew.-%.

Beispiel C

Phytophthora-Test (Tomate) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen viele der erfindungsgemäßen Verbindungen bei 10 ppm.

Beispiel D

Venturia-Test (Apfel) protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung. Eine deutliche Überlegenheit in der Wirksamkeit gegenüber dem Stand der Technik zeigen die Verbindungen bei einer Wirkstoffkonzentration von 10 ppm.

Herstellungsbeispiele

Beispiel 1

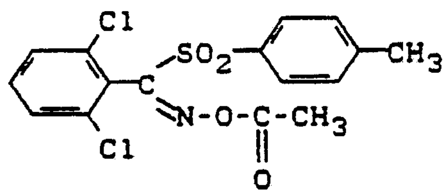

25,8 g (0,075 m) 2,6-Dichlor-α-(4-methyl-phenylsulfonyl)-benzaldoxim werden mit 100 ml Acetanhydrid 24 Stunden auf 70-80°C erhitzt. Das Reaktionsgemisch wird dann eingeengt, der Rückstand heiß mit Isopropanol verrührt, heiß abgesaugt, gewaschen und getrocknet. Man erhält 23,8 g (82 % der Theorie) an gewünschtem Produkt mit einem Schmelzpunkt von 179°C.

Analog werden Verbindungen der Formel (I)

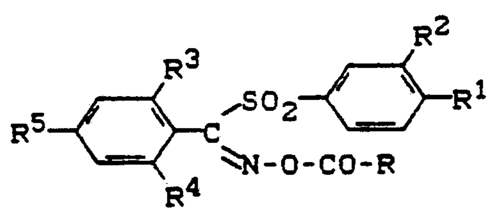

(I)

hergestellt:

| Beispiel-Nr. | R | $R^1$ | $R^2$ | $R^3$ | $R^4$ | $R^5$ | Physikalische Daten [Schmelzpunkt °C] |
|---|---|---|---|---|---|---|---|
| 2 | $-OC_3H_7-i$ | $-CH_3$ | H | Cl | Cl | H | 201 |
| 3 | $-OC_3H_7-i$ | H | H | Cl | Cl | H | 158 |
| 4 | $-OC_3H_7-i$ | Cl | H | Cl | Cl | H | 174 |
| 5 | $-OCH_3$ | $-CH_3$ | H | Cl | Cl | H | 178 |
| 6 | $-OC_2H_5$ | $-CH_3$ | H | Cl | Cl | H | 185 |
| 7 | $-OCH_2-CH=CH_2$ | $-CH_3$ | H | Cl | Cl | H | 158 |
| 8 | $-OCH_3$ | Cl | H | Cl | Cl | H | 164 |
| 9 | $-OC_2H_5$ | Cl | H | Cl | Cl | H | 142 |
| 10 | $-OCH_2-CH=CH_2$ | Cl | H | Cl | Cl | H | 133 |
| 11 | $-OCH_3$ | H | H | Cl | Cl | H | 112 |
| 12 | $-OC_2H_5$ | H | H | Cl | Cl | H | 137 |
| 13 | $-OCH_2-CH=CH_2$ | H | H | Cl | Cl | H | 120 |
| 14 | $-OC_4H_9-i$ | H | H | Cl | Cl | H | 138 |
| 15 | $-OC_4H_9-i$ | $-CH_3$ | H | Cl | Cl | H | 183 |
| 16 | $-OC_2H_5$ | $-CH_3$ | H | H | Cl | H | 127 |
| 17 | $-OCH_3$ | $-CH_3$ | H | H | Cl | H | 133 |
| 18 | $-OC_3H_7-i$ | $-CH_3$ | H | H | Cl | H | 113 |
| 19 | $-OCH_2-CH=CH_2$ | $-CH_3$ | H | H | Cl | H | 103 |
| 20 | $-OC_2H_5$ | $-CH_3$ | H | F | F | H | 196 |
| 21 | $-OC_2H_5$ | Cl | H | F | F | H | 131 |
| 22 | $-OCH_2-C_6H_5$ | $-CH_3$ | H | H | Cl | H | 125 |
| 23 | $-OC_4H_9-i$ | $-CH_3$ | H | H | Cl | H | 100 |
| 24 | $-OC_2H_5$ | H | H | F | F | H | 168 |
| 25 | $-OC_2H_5$ | F | H | Cl | Cl | H | 173 |
| 26 | $-OC_2H_5$ | $-OCH_3$ | H | Cl | Cl | H | 157 |
| 27 | $-OC_2H_5$ | H | $-CF_3$ | Cl | Cl | H | 112 |

| Beispiel-Nr. | R | $R^1$ | $R^2$ | $R^3$ | $R^4$ | $R^5$ | Physikalische Daten [Schmelzpunkt °C] |
|---|---|---|---|---|---|---|---|
| 28 | $-OCH_3$ | F | H | Cl | Cl | H | 171 |
| 29 | $-OC_3H_7$-i | F | H | Cl | Cl | H | 165 |
| 30 | $-OCH_2-CH=CH_2$ | F | H | Cl | Cl | H | 127 |
| 31 | $-OC_2H_5$ | F | H | F | F | H | 172 |
| 32 | $-OCH_2-CCl_3$ | $-CH_3$ | H | H | Cl | H | 149 |
| 33 | 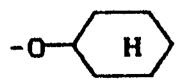 | $-CH_3$ | H | H | Cl | H | 147 |
| 34 | $-OC_2H_5$ | $-OCH_3$ | H | Cl | F | H | 143 |
| 35 | $-CH_3$ | H | H | Cl | Cl | H | 152 |
| 36 | $-CH_3$ | Cl | H | Cl | Cl | H | 170 |
| 37 | $-CH_2Cl$ | $-CH_3$ | H | Cl | Cl | H | 172 |
| 38 | $-CH_2Cl$ | H | H | Cl | Cl | H | 169 |
| 39 | $-CH_3$ | $-CH_3$ | H | H | Cl | H | 103 |
| 40 | 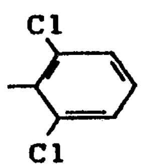 | -Cl | H | Cl | Cl | H | 200-202 |
| 41 | 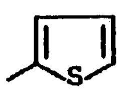 | Cl | H | Cl | Cl | H | 178-180 |

| Beispiel-Nr. | R | $R^1$ | $R^2$ | $R^3$ | $R^4$ | $R^5$ | Physikalische Daten [Schmelzpunkt °C] |
|---|---|---|---|---|---|---|---|
| 42 | 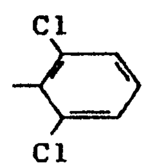 | $-CH_3$ | H | Cl | Cl | H | 203-205 |
| 43 | 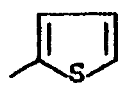 | $-CH_3$ | H | Cl | Cl | H | 173-175 |
| 44 | $-OCH_3$ | $-CH_3$ | H | Cl | F | H | 172 |
| 45 | $-OC_2H_5$ | $-CH_3$ | H | Cl | F | H | 192 |
| 46 | $-OC_3H_7$-i | $-CH_3$ | H | Cl | F | H | 161 |
| 47 | $-OC_4H_9$-i | $-CH_3$ | H | Cl | F | H | 169 |
| 48 | $-OCH_2-CH=CH_2$ | $-CH_3$ | H | Cl | F | H | 147 |
| 49 | $-OCH_2-CCl_3$ | $-CH_3$ | H | Cl | F | H | 173 |
| 50 | $-OC_2H_5$ | Cl | H | Cl | F | H | 148 |
| 51 | $-OCH_2-CH=CH_2$ | $-CH_3$ | H | H | Cl | Cl | 75 |
| 52 | $-OC_2H_5$ | $-CH_3$ | H | H | Cl | Cl | 77 |
| 53 | $-OCH_3$ | $-CH_3$ | H | H | Cl | Cl | |
| 54 | $-OC_3H_7$-i | $-CH_3$ | H | H | Cl | Cl | |
| 55 | $-OC_4H_9$-i | Cl | H | Cl | Cl | H | 145 |
| 56 | $-CH_2Cl$ | Cl | H | Cl | Cl | H | 132 |
| 57 | $-O-C_6H_5$ | $-CH_3$ | H | Cl | Cl | H | |

| Beispiel-Nr. | R | $R^1$ | $R^2$ | $R^3$ | $R^4$ | $R^5$ | Physikalische Daten [Schmelzpunkt °C] |
|---|---|---|---|---|---|---|---|
| 58 | $-OCH_3$ | $-CH_3$ | H | F | F | H | 175 |
| 59 | $-CH_3$ | $-CH_3$ | H | F | F | H | 148 |
| 60 | $-O-CH_2-CH=CH_2$ | $-CH_3$ | H | F | F | H | 140 |
| 61 | $-OCH_2CCl_3$ | $-CH_3$ | H | F | F | H | 128 |
| 62 | 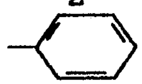 | Cl | H | Cl | Cl | H | 206 |
| 63 | 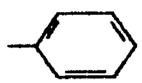 | $CH_3$ | H | Cl | Cl | H | 193 |
| 64 | 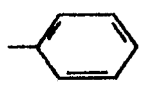 | $CH_3$ | H | H | Cl | H | 120 |
| 65 | $-CH_2Cl$ | $CH_3$ | H | H | Cl | H | 123 |
| 66 | $-OCH_3$ | H | H | H | Cl | H | 145 |
| 67 | $-OC_4H_9-i$ | H | H | H | Cl | H | 122 |
| 68 | $-OC_2H_5$ | H | H | H | Cl | H | 107 |
| 69 | $-OCH_2CCl_3$ | H | H | H | Cl | H | 120 |
| 70 | $-O-CH_2-CH=CH_2$ | H | H | H | Cl | H | 64 |
| 71 | $-OCH_3$ | Cl | H | H | Cl | H | 145-47 |
| 72 | $-OC_4H_9-i$ | Cl | H | H | Cl | H | 122 |
| 73 | $-OC_2H_5$ | Cl | H | H | Cl | H | 121 |
| 74 | $-OCH_2CCl_3$ | Cl | H | H | Cl | H | 124 |
| 75 | $-O-CH_2-CH=CH_2$ | Cl | H | H | Cl | H | 104 |

| Beispiel-Nr. | R | $R^1$ | $R^2$ | $R^3$ | $R^4$ | $R^5$ | Physikalische Daten [Schmelzpunkt °C] |
|---|---|---|---|---|---|---|---|
| 76 | $-OCH_3$ | Cl | H | Cl | F | H | 160 |
| 77 | $-O-CH_2-CH=CH_2$ | Cl | H | Cl | F | H | 104 |
| 78 | $-OC_4H_9-i$ | Cl | H | Cl | F | H | 109 |
| 79 | $-OCH_2CCl_3$ | Cl | H | Cl | F | H | 171 |
| 80 | $-CH_3$ | Cl | H | H | Cl | H | 131 |
| 81 | $-CH_2Cl$ | Cl | H | H | Cl | H | 108 |
| 82 | 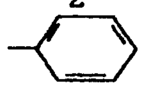 | Cl | H | H | Cl | H | 112 |
| 83 | $-CH_3$ | H | H | H | Cl | H | 112 |
| 84 | $-CH_2Cl$ | H | H | H | Cl | H | 77 |
| 85 | 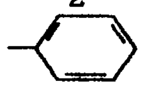 | H | H | H | Cl | H | 113 |
| 86 | $-O-CH_2$ 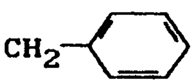 | H | H | H | Cl | H | 101 |
| 87 | $-O-CH_2$ 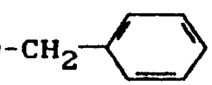 | Cl | H | H | Cl | H | 77 |
| 88 | $-O-CH_2$ 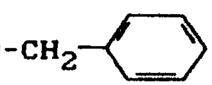 | Cl | H | F | Cl | H | 126 |

Herstellung der Ausgangsprodukte

Beispiel 1A

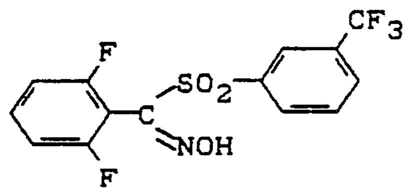

19,2 g (0,1 m) α-Chlor-2,6-difluor-benzaldoxim werden in 200 ml Methanol gelöst und mit 23,2 g (0,1 m) 3-Trifluormethylbenzolsulfinsaurem Natriumsalz bei Zimmertemperatur versetzt. Das Reaktionsgemisch wird ca. 15 Stunden bei dieser Temperatur gehalten, anschließend auf 1 l Eiswasser gegossen, ausgerührt, abgesaugt, gewaschen und getrocknet. Das Reaktionsprodukt wird aus Toluol umkristallisiert. Man erhält 24 g (62,5 % der Theorie) α-3-Trifluormethylphenylsulfonyl-2,6-difluorbenzaldoxim vom Schmelzpunkt 127°C.

Analog können die Verbindungen der Formel (II)

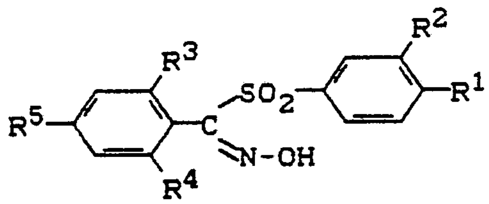

(II)

hergestellt werden.

| Bsp. | | $R^1$ | $R^2$ | $R^3$ | $R^4$ | $R^5$ | Physik.Daten (Schmelzpt.°C) |
|---|---|---|---|---|---|---|---|
| 2 | A | $CH_3$ | H | H | Cl | Cl | 143 |
| 3 | A | Cl | H | Cl | F | H | 158 |
| 4 | A | $CH_3$ | H | Cl | F | H | 170 |
| 5 | A | H | H | Cl | F | H | 143 |
| 6 | A | $OCH_3$ | H | Cl | F | H | 145 |
| 7 | A | $CH_3$ | H | H | Cl | H | 147 |
| 8 | A | H | H | Cl | Cl | H | 148 |
| 9 | A | F | H | Cl | Cl | H | 154 |
| 10 | A | Cl | H | Cl | Cl | H | 111 |
| 11 | A | H | H | F | F | H | 149 |
| 12 | A | Cl | H | F | F | H | 171 |
| 13 | A | $CH_3$ | H | F | F | H | 166 |
| 14 | A | $OCH_3$ | H | Cl | Cl | H | 163 |
| 15 | A | $-OCH_3$ | H | F | F | H | 145 |
| 16 | A | $-OCH_3$ | H | Cl | H | H | 141 |
| 17 | A | H | $CF_3$ | Cl | Cl | H | 148 |

## Ansprüche

1. Benzaldoxim-Derivate der allgemeinen Formel (I)

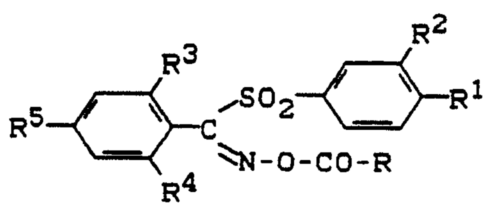

(I)

in welcher
R für Alkyl, Alkoxy, Halogenalkyl, Halogenalkoxy, Alkenyloxy, gegebenenfalls jeweils ein-bis mehrfach, gleich oder verschieden substituiertes Aryl oder Aryloxy, gegebenenfalls ein-bis mehrfach, gleich oder verschieden im Arylrest substituiertes Aralkyloxy, für gegebenenfalls ein-bis mehrfach, gleich oder verschieden substituiertes Cycloalkyloxy oder für einen gegebenenfalls einbis mehrfach, gleich oder verschieden substituierten Heterocyclus steht,
$R^1$ für Wasserstoff, Halogen, Alkyl oder Alkxoy steht,
$R^2$ für Wasserstoff, Alkyl oder Halogenalkyl steht,
$R^3$ für Wasserstoff oder Halogen steht,
$R^4$ für Halogen steht und
$R^5$ für Wasserstoff oder Halogen steht, deren Isomere und Isomerengemische.

2. Benzaldoxim-Derivate gemäß Anspruch 1, worin in der Formel (I)
R für geradkettiges oder verzweigtes Alkyl mit 1 bis 6 Kohlenstoffatomen, für geradkettiges oder verzweigtes Alkoxy mit 1 bis 6 Kohlenstoffatomen, für Halogenalkyl oder Halogenalkoxy mit jeweils 1 bis 6 Kohlenstoffatomen im geradkettigen oder verzweigten Halogenalkylrest und 1 bis 9 gleichen oder verschiedenen Halogenatomen, für geradkettiges oder verzweigtes Alkenyloxy mit 2 bis 6 Kohlenstoffatomen, für unsubstituiertes oder jeweils ein-bis fünffach, gleich oder verschieden durch geradkettiges oder verzweigtes Alkyl mit 1 bis 4 Kohlenstoffatomen, geradkettiges oder verzweigtes Alkoxy mit 1 bis 4 Kohlenstoffatomen, Halogen, Nitro und Acetyl substituiertes Aryl oder Aryloxy mit 6 bis 10 Kohlenstoffatomen, für unsubstituiertes oder ein-bis fünffach, gleich oder verschieden durch geradkettiges oder verzweigtes Alkyl oder Alkoxy mit 1 bis 4 Kohlenstoffatomen, Halogen, Nitro und Acetyl substituiertes Aralkyloxy mit 6 bis 12 Kohlenstoffatomen im Arylrest und 1 bis 6 Kohlenstoffatomen im geradkettigen oder verzweigten Alkylrest, für unsubstituiertes oder ein-bis fünffach, gleich oder verschieden durch geradkettiges oder verzweigtes Alkyl mit 1 bis 4 Kohlenstoffatomen substituiertes Cycloalkyloxy mit 5 bis 7 Kohlenstoffatomen im Cycloalkylteil oder für einen unsubstituierten oder ein-bis dreifach, gleich oder verschieden durch geradkettiges oder verzweigtes Alkyl mit 1 bis 4 Kohlenstoffatomen substituierten, gesättigten, teilweise oder ganz ungesättigten Heterocyclus mit 5 bis 8 Ringgliedern steht, der 1 bis 3 Heteroatome enthalten kann;
R' für Wasserstoff, Halogen, geradkettiges oder verzweigtes Alkyl mit 1 bis 6 Kohlenstoffatomen oder geradkettiges oder verzweigtes Alkoxy mit 1 bis 6 Kohlenstoffatomen steht,
$R^2$ für Wasserstoff, geradkettiges oder verzweigtes Alkyl mit 1 bis 6 Kohlenstoffatomen oder für geradkettiges oder verzweigtes Halogenalkyl mit 1 bis 6 Kohlenstoffatomen und 1 bis 9 gleichen oder verschiedenen Halogenatomen steht,
$R^3$ für Wasserstoff oder Halogen steht,
$R^4$ für Halogen steht und
$R^5$ für Wasserstoff oder Halogen steht, deren Isomeren und Isomerengemische.

3. Benzaldoxim-Derivate gemäß Anspruch 1, worin in der Formel (I)
R für geradkettiges oder verzweigtes Alkyl mit 1 bis 4 Kohlenstoffatomen, für geradkettiges oder verzweigtes Alkoxy mit 1 bis 4 Kohlenstoffatomen, für Halogenalkyl oder Halogenalkoxy mit jeweils 1 bis 4 Kohlenstoffatomen im geradkettigen oder verzweigten Halogenalkylrest und 1 bis 5 gleichen oder verschiedenen Halogenatomen, für geradkettiges oder verzweigtes Alkenyloxy mit 2 bis 5 Kohlenstoffatomen, für unsubstituiertes oder jeweils ein-bis fünffach, gleich oder verschieden durch geradkettiges oder verzweigtes Alkyl mit 1 bis 3 Kohlenstoffatomen, geradkettiges oder verzweigtes Alkoxy mit 1 bis 3 Kohlenstoffatomen, Halogen, Nitro und Acetyl substituiertes Phenyl oder Phenoxy, für unsubstituiertes oder ein-bis dreifach, gleich oder verschieden durch geradkettiges oder verzweigtes Alkyl oder Alkoxy mit 1 bis 3 Kohlenstoffatomen, Halogen, Nitro und Acetyl substi tuiertes Benzyloxy, Phenethyloxy oder Phenylpropyloxy, und für unsubstituiertes oder ein-bis dreifach, gleich oder verschieden durch geradkettiges oder verzweigtes Alkyl mit 1 bis 3 Kohlenstoffatomen substituiertes Cycloalkyloxy mit 5 bis 7 Kohlenstoffatomen im Cycloalkylteil oder für einen unsubstituierten oder einfach durch geradkettiges oder verzweigtes Alkyl mit 1 bis 3 Kohlenstoffatomen substituierten, gesättigten, teilweise gesättigten oder ungesättigten Heterocyclus mit 5 bis 7 Ringgliedern steht, der 1 oder 2 Schwefel-, Stickstoff-und/oder Sauerstoffatome enthalten kann;
$R^1$ für Wasserstoff, Halogen, geradkettiges oder verzweigtes Alkyl mit 1 bis 4 Kohlenstoffatomen oder geradkettiges oder verzweigtes Alkoxy mit 1 bis 4 Kohlenstoffatomen steht,
$R^2$ für Wasserstoff, geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 5 gleichen oder verschiedenen Halogenatomen steht,
$R^3$ für Wasserstoff oder Halogen steht,
$R^4$ für Halogen steht und
$R^5$ für Wasserstoff oder Halogen steht, deren Isomere und Isomerengemische.

4. Benzaldoxim-Derivate gemäß Anspruch 1, worin in der Formel (I)
R für geradkettiges oder verzweigtes Alkyl mit 1 bis 6 Kohlenstoffatomen, für geradkettiges oder verzweigtes Alkoxy mit 1 bis 6 Kohlenstoffatomen, für Halogenalkyl oder Halogenalkoxy mit jeweils 1 oder 2 Kohlenstoffatomen im Halogenalkylrest und 1 bis 4 gleichen oder verschiedenen Halogenatomen, für geradkettiges oder verzweigtes Alkenyloxy mit 2 oder 3 Kohlenstoffatomen, für unsubstituiertes oder jeweils ein-oder zweifach, gleich oder verschieden durch Alkyl mit 1 oder 2 Kohlenstoffatomen, Alkoxy mit 1 oder 2 Kohlenstoffatomen, Halogen, Nitro und Acetyl substituiertes Phenyl oder Phenoxy, für unsubstituiertes oder ein-bis dreifach, gleich oder verschieden durch Alkyl oder Alkoxy mit 1 oder 2 Kohlenstoffatomen oder Halogen substituiertes Benzyl, für
unsubstituiertes oder ein-bis dreifach, gleich oder verschieden durch Alkyl mit 1 oder 2 Kohlenstoffatomen substituiertes Cycloalkyloxy mit 5 oder 6 Kohlenstoffatomen im Cycloalkylteil oder für einen unsubstituierten

oder ein-oder zweifach, gleich oder verschieden durch Alkyl mit 1 oder 2 Kohlenstoffatomen substituierten, ungesättigten Heterocyclus mit 5 oder 6 Ringgliedern steht, der Stickstoff-und/oder ein Schwefelatom enthalten kann;

$R^1$ für Wasserstoff, Halogen, Alkyl mit 1 oder 2 Kohlenstoffatomen oder Alkoxy mit 1 oder 2 Kohlenstoffatomen steht,

$R^2$ für Wasserstoff, Alkyl mit 1 oder 2 Kohlenstoffatomen oder für Halogenalkyl mit 1 oder 2 Kohlenstoffatomen und 1 bis 3 gleichen oder verschiedenen Halogenatomen steht,

$R^3$ für Wasserstoff oder Halogen steht,

$R^4$ für Halogen steht und

$R^5$ für Wasserstoff oder Halogen steht, deren Isomere und Isomerengemische.

5. Benzaldoxim-Derivate gemäß Anspruch 1, wobei in der Formel (I)

R für Methyl, Ethyl, Methoxy, Ethoxy, n-Propoxy, iso-Propoxy, iso-Butoxy, Allyloxy, 2,2,2-Trichlorethoxy, Benzyloxy, Chlormethyl, Phenyl, 2,6-Dichlorphenyl, 2,4-Dichlorphenyl, Phenoxy, Thienyl oder Cyclohexyloxy steht,

$R^1$ für Wasserstoff, Fluor, Chlor, Methyl oder Methoxy steht,

$R^2$ für Wasserstoff oder Trifluormethyl steht,

$R^3$ für Wasserstoff Fluor, oder Chlor steht,

$R^4$ für Fluor oder Chlor steht und

$R^5$ für Wasserstoff oder Chlor steht, deren Isomere und Isomerengemische.

6. Verfahren zur Herstellung von Benzaldoxim-Derivaten der Formel (I)

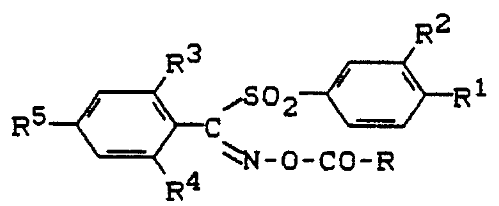

(I)

in welcher

R für Alkyl, Alkoxy, Halogenalkyl, Halogenalkoxy, Alkenyloxy, gegebenenfalls jeweils ein-bis mehrfach, gleich oder verschieden substituiertes Aryl oder Aryloxy, gegebenenfalls ein-bis mehrfach, gleich oder verschieden im Arylrest substituiertes Aralkyloxy, für gegebenenfalls ein-bis mehrfach, gleich oder verschieden substituiertes Cycloalkyloxy oder für einen gegebenenfalls ein-bis mehrfach, gleich oder verschieden substituierten Heterocyclus steht,

$R^1$ für Wasserstoff, Halogen, Alkyl oder Alkoxy steht,

$R^2$ für Wasserstoff, Alkyl oder Halogenalkyl steht,

$R^3$ für Wasserstoff oder Halogen steht,

$R^4$ für Halogen steht und

$R^5$ für Wasserstoff oder Halogen steht, deren Isomere und Isomerengemische dadurch gekennzeichnet, daß man Phenylsulfonyl-benzaldoxime der allgemeinen Formel (II)

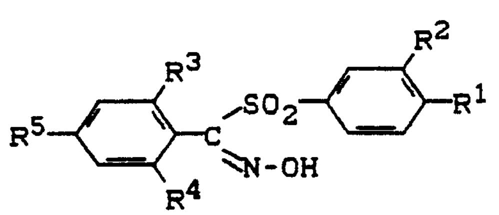

(II)

in welcher

$R^1$, $R^2$, $R^3$, $R^4$ und $R^5$ die oben angegebenen Bedeutungen haben,

mit Carbonylverbindungen der allgemeinen Formel (III)

$$X - CO - R \qquad (III)$$

in welcher

R die oben angegebenen Bedeutungen hat und

X für ein Halogenatom, oder für den Rest -O-COR steht, worin R die oben angegebene Bedeutung hat, gegebenenfalls in Gegenwart eines Lösungs-oder Verdünnungsmittels und gegebenenfalls in Gegenwart eines Säurebindemittels umsetzt.

7. Schädlingsbekämpfungsmittel, gekennzeichnet durch einen Gehalt an mindestens einem Benzaldoxim-Derivat der Formel (I) nach den Ansprüchen 1 und 6.

8. Verwendung von Benzaldoxim-Derivaten der Formel (I) nach den Ansprüchen 1 und 6 zur Bekämpfung von Schädlingen.

9. Verfahren zur Bekämpfung von Schädlingen, dadurch gekennzeichnet, daß man Benzaldoxim-Derivate der Formel (I) nach den Ansprüchen 1 und 6 auf Schädlinge und/oder ihren Lebensraum einwirken läßt.

10. Verfahren zur Herstellung von Schädingsbekämpfungsmitteln, dadurch gekennzeichnet, daß man Benzaldoxim-Derivate der Formel (I) nach den Ansprüchen 1 und 6 mit Streckmitteln und/oder oberflächenaktiven Mitteln vermischt.

Europäisches Patentamt

# EUROPÄISCHER RECHERCHENBERICHT

Nummer der Anmeldung

EP 88103146.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A1 - 0 205 076 (BAYER)<br>* Ansprüche 1,6-10 *<br>-- | 1,6-10 | C 07 C 147/06<br>A 01 N 41/10 |
| A | US - A - 4 382 893 (ELMAR STURM et al.)<br>* Anspruch 1 *<br>-- | 1 | |
| D,A | CH - A - 423 350 (SHELL RESEARCH)<br>* Patentanspruch *<br>---- | 1,7 | |
| | | | RECHERCHIERTE SACHGEBIETE (Int. Cl.4) |
| | | | C 07 C 147/00<br>C 07 C 131/00 |

Der vorliegende Recherchenbericht wurde für alle Patentansprüche erstellt.

| Recherchenort | Abschlußdatum der Recherche | Prüfer |
|---|---|---|
| WIEN | 14-06-1988 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument